# EUROPEAN PATENT SPECIFICATION

(11) **EP 3 328 397 B1**
(45) Date of publication and mention of the grant of the patent: **12.07.2023**
(21) Application number: 16833569.3
(22) Date of filing: 28.07.2016
(51) Int. Cl.: A61K 35/28, C12N 5/0775, A61P 1/02, A61P 17/02, A61P 19/04

(54) **EXOSOME COMPOSITIONS AND METHODS FOR PREPARATION AND USE THEREOF FOR REGULATING AND CONDITIONING SKIN AND HAIR**
EXOSOMZUSAMMENSETZUNGEN UND VERFAHREN ZUR HERSTELLUNG UND VERWENDUNG DAVON ZUR REGULIERUNG UND PFLEGE VON HAUT UND HAAR
COMPOSITIONS D'EXOSOMES ET MÉTHODES POUR LA PRÉPARATION ET L'UTILISATION DE CES DERNIERS POUR LA RÉGULATION ET LE CONDITIONNEMENT DE LA PEAU ET DES CHEVEUX

(30) Priority: 31.07.2015 US 201562199696 P
(43) Date of publication of application: 06.06.2018
(73) Proprietor: Exotropin, LLC, New York, NY 10036 (US)
(72) Inventor: LUDLOW, John, W., NC 27709 (US); BUEHRER, Benjamin, NC 27709 (US); PIERACCINI, Peter, Durham, NC 27707 (US)
(74) Representative: Gilani, Anwar
(86) International application number: PCT/US2016/044458
(87) International publication number: WO 2017/023690

(56) References cited:
- WO-A1-2014/159662
- US-A1- 2011 003 008
- US-A1- 2011 004 169
- US-A1- 2011 301 090
- US-A1- 2015 023 908
- US-A1- 2015 024 011
- DATABASE BIOSIS [Online] BIOSCIENCES INFORMATION SERVICE, PHILADELPHIA, PA, US; April 2015 (2015-04), LUDLOW J ET AL: "Mesenchymal Stem Cell Exosomes - Potential Regenerative Medicine Application for Tendinopathy", XP002788942, Database accession no. PREV201600122429 & FASEB JOURNAL, vol. 29, no. Suppl. 1, April 2015 (2015-04), page 1018.3, EXPERIMENTAL BIOLOGY MEETING 2015; BOSTON, MA, USA; MARCH 28 -APRIL 01, 2015 ISSN: 0892-6638(print)
- BASU J & LUDLOW J: "Wound Healing, Skin Regeneration and Tendon Repair with MSC-sourced Exosomes: Functional Evaluation In Vitro and In Vivo and Methods of Manufacture", TISSUE ENGINEERING PART A, vol. 21, no. Suppl. 1, September 2015 (2015-09), pages S112-S113, XP055557089, & 4th World Congress of the Tissue Engineering and Regenerative Medicine International Society (TERMIS); Boston, MA, USA; 8-11 September 2015
- BASU J & LUDLOW J: "Exosomes for repair, regeneration and rejuvenation", EXPERT OPINION ON BIOLOGICAL THERAPY, vol. 16, no. 4, 28 January 2016 (2016-01-28), pages 489-506, XP055557079, ISSN: 1471-2598, DOI: 10.1517/14712598.2016.1131976

## Description

### TECHNICAL FIELD

The present disclosure relates to stem cell exosome compositions, and preparation thereof, for uses including regulating and conditioning skin and hair.

### BACKGROUND

Existing treatments for aging and wrinkled skin are temporary and many treatments are ineffective or have unwanted side effects. During the aging process, skin loses thickness and resiliency due to a loss of collagen and other elastic proteins in the dermal layers. These losses can result in fine lines and wrinkles. Common non-invasive methods for treating fine lines and wrinkles include application of formulations topically to the skin. The formulations commonly include alpha and beta hydroxyl acids, retinoic acids, argirelines, and vitamins. None of these formulations completely eliminate wrinkles and many are expensive. In addition, while some formulations irritate the skin to elicit a wound healing response, this does not result in replenishment of the thinning skin to sufficiently treat and/or prevent age-related defects.

Skin aging is characterized by a decrease in collagen synthesis and an increase in collagen breakdown. It is generally accepted that the breakdown of collagen is mediated by metalloproteinases (1). The loss in dermal collagen is believed to contribute to the appearance of fine lines and wrinkles. It is believed that biological factors that stimulate collagen production in wound healing might provide a benefit for aging skin. As a result, formulations for regulating skin condition such as those for treating and/or reducing the appearance of fine lines and wrinkles can include growth factors, peptide fragments, and other biologically active molecules.

Growth factors are typically peptides with diverse biological effects. Some growth factor families that have been identified as useful in wound healing and epidermal remodeling include, e.g., transforming growth factor-β (TGF-β), epidermal growth factor (EGF), insulin-like growth factors (IGFs), platelet-derived growth factor (PDGF), and fibroblast growth factors (FGFs). One source of growth factors for regulating skin condition includes those secreted by cultured living cells. The growth factors and other extracellular molecules including proteins and peptides are secreted into the nutrient medium in which they are cultured. Medium exposed to cells in culture is referred to as "conditioned medium."

In addition to secreting extracellular proteins such as growth factors, cultured cells also secrete extracellular vesicles known as microvesicles or exosomes. Once thought of as contaminating debris in cell culture, these secreted microvesicles that are also called exosomes are packed with protein and RNA cargos. Exosomes contain functional mRNA, miRNA, DNA, and protein molecules that can be taken up by target cells. Proteomic and genomic analysis of exosome cargo has revealed a broad range of signaling factors that are both cell type-specific as well as differentially regulated based on the secreting cells' environment [2]. HSP70 has been previously shown to be a cargo constituent of exosomes [3, 4, 5]. The genetic information contained in exosomes may influence or even direct the fate of the target cell, for example by triggering target cell activation, migration, growth, differentiation or de-differentiation, or by promoting apoptosis or necrosis. As such, exosomes may provide additional cell factors for assistance in wound healing and epithelial remodeling.

Stem cell therapies also represent a compelling means for repairing damaged tissue, and several of these strategies have been evaluated for repair of oral tissues and craniomaxillofacial bone [6-8]. For example, mesenchymal stem cells (MSCs) represent an accessible, numerous and well-characterized source of stem cells. A range of studies have examined the ability of stem cells to regenerate periodontal tissues, with studies including stem cells derived from adipose tissue and bone marrow [9, 10]. However, while these reports support the potential for stem cell based therapeutics in gingivitis and periodontitis, none are yet commercially available.

Despite repeated demonstration of MSC-induced improvements in the repair of tissues such as bone, cartilage and tendon, a consensus mechanism for MSC-induced repair remains elusive. The intuitive concept that therapeutic stem cells engraft and differentiate at sites of tissue damage is not well supported given the low numbers of cells retained over time at *in vivo* injection sites, with a number of encapsulation and delivery technologies such as microbeads and cell sheets under development [11, 12]. Alternatively, MSCs have been shown to exert tissue repair effects through a paracrine modality, secreting factors that trigger host-site damage repair cascades [13-15]. Periodontal ligament cells have also been shown to proliferate in response to conditioned media derived from stem cells [16]. In addition, environmental factors such as pro-inflammatory cytokines and platelet lysate have been shown to stimulate changes in MSC paracrine factor composition and abundance [17, 18]. Concomitant with growing interest in MSC paracrine activity, MSC-derived exosomes have become a relatively new target for investigation [19]. The hypothesis that exosomes exert the primary paracrine activities of stem cells has garnered support through *in vivo* tissue repair models [20, 21].

Thus, an unmet need remains for more effective topical formulations for regulating skin condition such as improving skin damage, wrinkles, and other defects including scars, keloids, skin discolorations, and skin abrasions.

The presently disclosed subject matter provides improved exosome compositions, and methods of preparation and use thereof, for regulating skin condition.

### SUMMARY

In one embodiment, a topical composition for regulating a cosmetic skin or hair condition is provided, the composition comprising: i) an effective amount of isolated exosomes having increased levels of heat shock stress-response molecules; and ii) a carrier, wherein the isolated exosomes are produced by a process comprising: (a) culturing stem cells in culture medium, wherein the culturing includes a step of heat shocking the stem cells in a serum-free culture medium by increasing the culture temperature to about 41°C to about 43 °C for about 1 hour to about 3 hours, and wherein the serum-free culture medium contains the exosomes having the increased levels of heat shock stress-response molecules; and (b) isolating the exosomes having increased levels of heat shock stress-response molecules from the serum-free medium.

In one embodiment, a method is provided for making a topical composition for regulating a cosmetic skin or hair condition, the method comprising combining an effective amount of isolated exosomes having increased levels of heat shock stress-response molecules with a carrier.

In one embodiment, a method is provided for regulating skin condition which comprises applying to human skin at least once a day over at least seven days a topical composition comprising: i) an effective amount of isolated stem cell exosomes having increased levels of heat shock stress-response molecules; and ii) a carrier, wherein the stem cell exosomes are produced by a process comprising: (a) culturing stem cells in culture medium, wherein the culturing includes a step of heat shocking the stem cells in a serum-free culture medium by increasing the culture temperature to about 41°C to about 43°C for about 1 hour to about 3 hours, and wherein the serum-free culture medium contains the exosomes having the increased levels of heat shock stress-response molecules; and (b) isolating the exosomes having increased levels of heat shock stress-response molecules from the serum-free medium, wherein regulating skin condition includes one or more of inducing increased skin integrity by cell renewal, enhancing water content or moisture of skin, reducing trans epidermal water loss, skin flaking, and scaling, improving skin thickness, enhancing skin tensile properties, reducing the appearance of dermal fine lines and wrinkles, improving skin texture, reducing skin pores size, enhancing skin smoothness, improving skin age spots, improving skin tone, or improving the appearance of scars and skin abrasions.

### BRIEF DESCRIPTION OF THE DRAWINGS

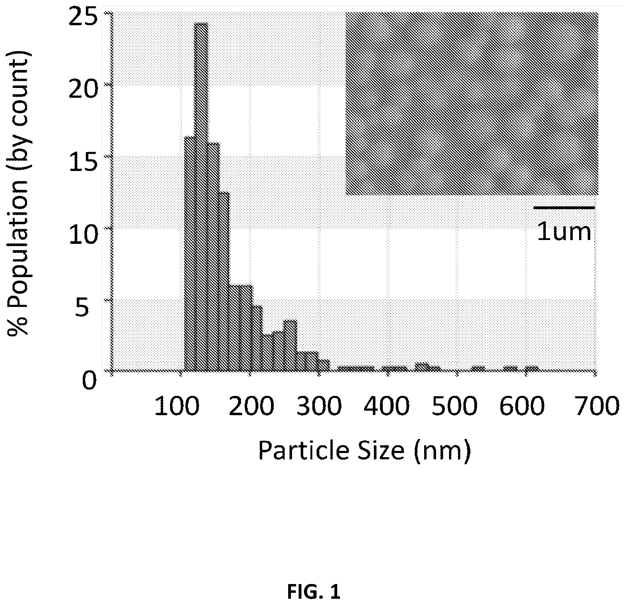

**FIG. 1** is a graph showing the size distribution (mean 152nm, mode 107nm) of a representative sample of isolated heat shock exosomes according to one or more embodiments of the present disclosure. The inset to FIG. 1 is a scanning electron microscopy image of a separate representative sample of the isolated heat shock exosomes according to one or more embodiments of the present disclosure showing the size and shape of the exosome particles.

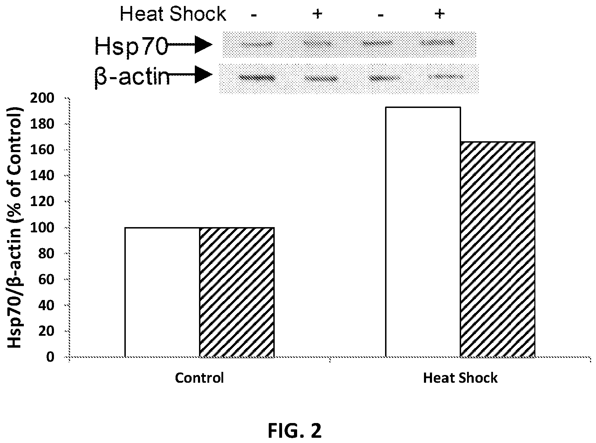

**FIG. 2** is a bar graph of quantified Western Blot data that shows the amount of HSP70 protein relative to β-actin protein in two separate preparations of exosomes: 1) secreted by cells cultured at 37°C without a heat shock step (Control; blank and hatched bars represent the separate preparations); and 2) secreted by cells subjected to a 2 hr heat shock step at 43°C (Heat Shock; blank and hatched bars represent the separate preparations), according to one or more embodiments of the present disclosure.

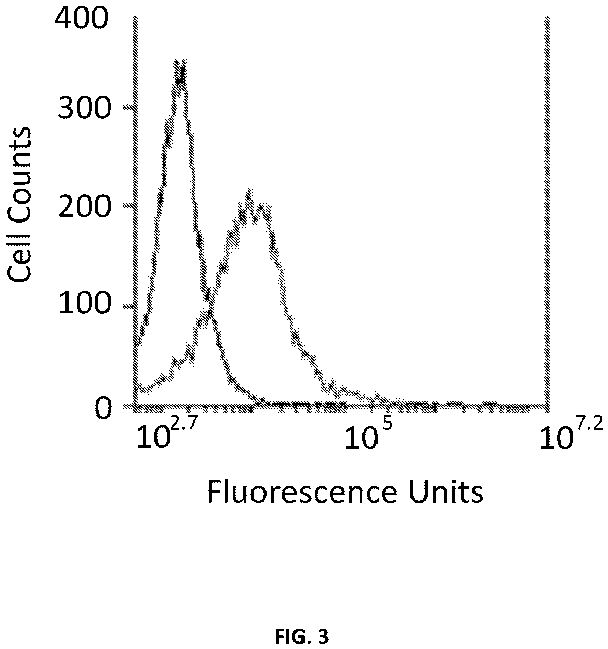

**FIG. 3** is a graph of histograms of flow cytometry data from HPAE cells incubated with isolated exosomes showing transfer of dye loaded into the exosomes to the HPAE cells. The HPAE cells were incubated with dye-loaded exosomes at 4°C (left-most histogram) or at 37°C (right-most histogram). The isolated exosomes were prepared from stem cells subjected to a heat shock step according to one or more embodiments of the present disclosure.

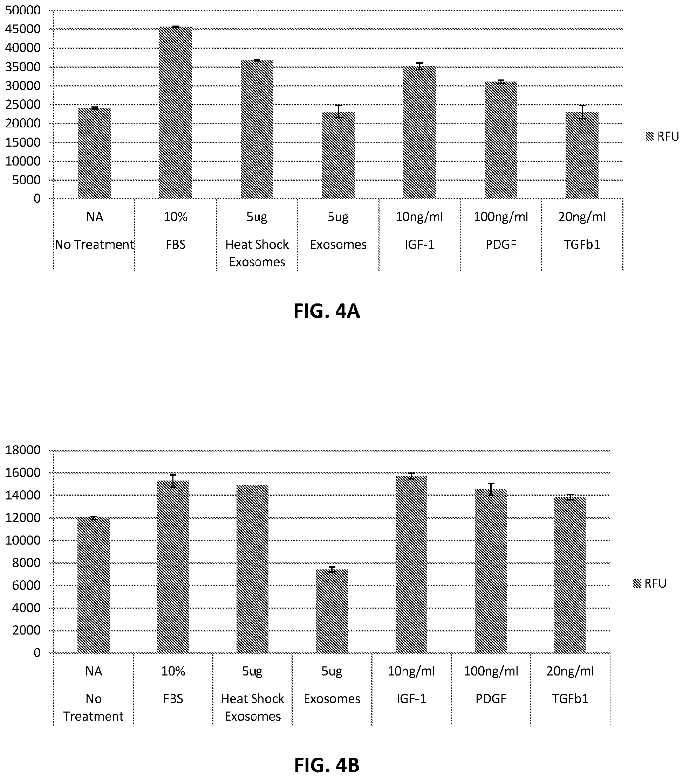

**FIG. 4A** is a graph showing the amount of cell proliferation in periodontal ligament fibroblasts after a 3 day incubation with serum free medium, various growth factors, or exosomes secreted from cells cultured with or without a heat shock step according to one or more embodiments of the present disclosure. Values shown on the Y axis are relative fluorescence units (RFU).
**FIG. 4B** is a graph showing the amount of cell proliferation in dermal fibroblasts after a 3 day incubation with serum free medium, various growth factors, or exosomes secreted from cells cultured with or without a heat shock step according to one or more embodiments of the present disclosure. Values shown on the Y axis are relative fluorescence units (RFU).

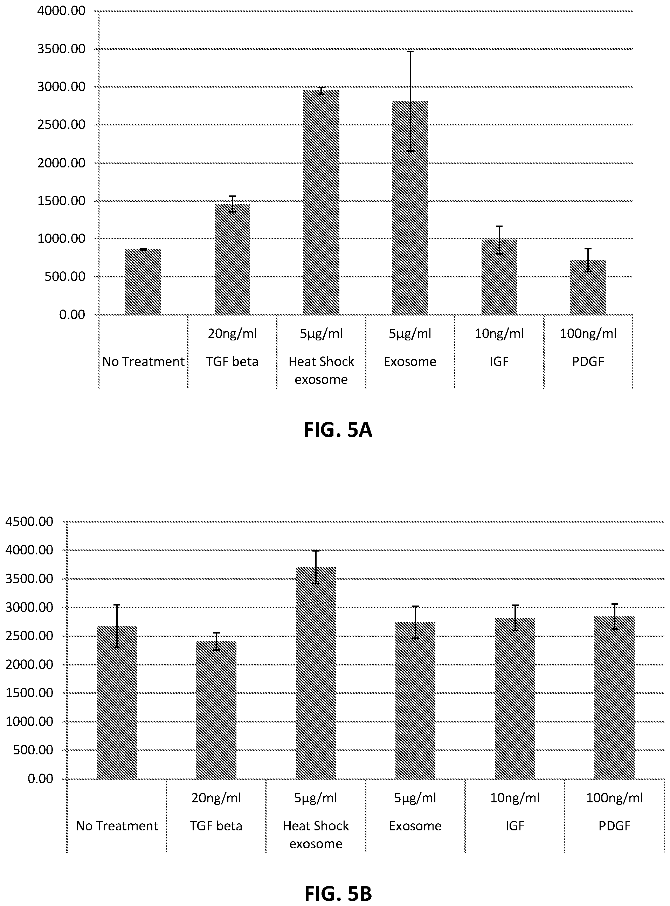

**FIG. 5A** is a graph showing the amount of collagen I production in periodontal ligament fibroblasts after a 48 hour incubation with medium control, various growth factors, or exosomes secreted from cells cultured with or without a heat shock step according to one or more embodiments of the present disclosure. Values shown on the Y axis are ng/ml of collagen.
**FIG. 5B** is a graph showing the amount of collagen I production in dermal fibroblasts after a 48 hour incubation with medium control, various growth factors, or exosomes secreted from cells cultured with or without a heat shock step according to one or more embodiments of the present disclosure. Values shown on the Y axis are ng/ml of collagen.

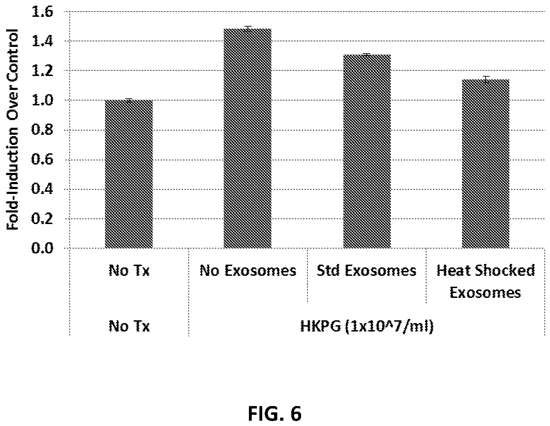

**FIG. 6** is a graph showing quantified RT-qPCR data of the inflammatory cytokine IL6 from periodontal ligament fibroblasts (PDLF) after being incubated overnight with the following treatments: without HKPG or exosomes (No Tx), with 10⁷/ml HKPG and without exosomes (No Exosomes), or with 10⁷/ml HKPG in combination with adipose stem cell-derived isolated exosomes prepared from cell cultures with a heat shock step (Heat shock Exosomes) and without a heat shock step (Std Exosomes), according to one or more embodiments of the present disclosure.

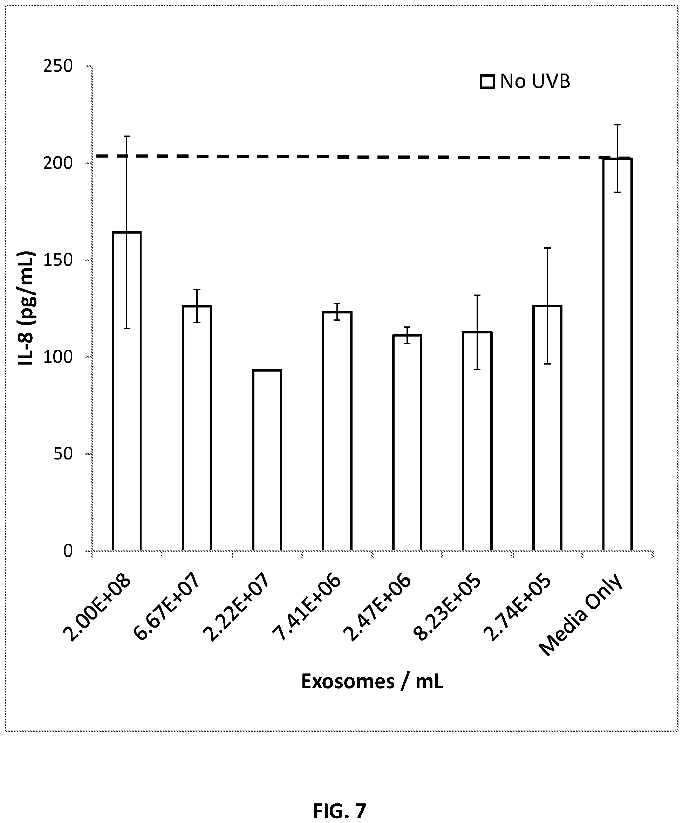

**FIG. 7** is a graph showing reduction in IL-8 production by human adult keratinocytes in the absence of UVB radiation (No UVB) with various amounts of the heat shock exosomes compared to a media control (Media Only) according to one or more embodiments of the present disclosure.

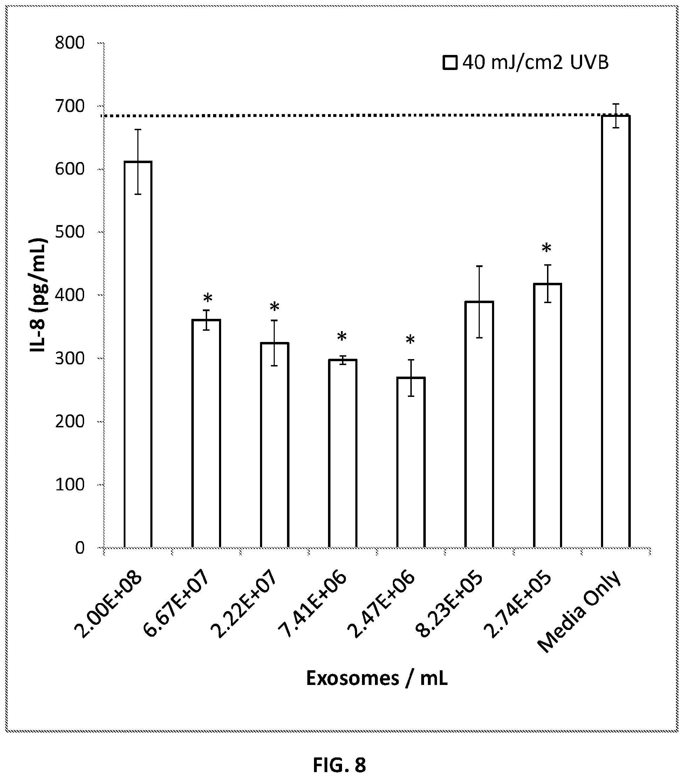

**FIG. 8** is a graph showing reduction in IL-8 production by human adult keratinocytes in the presence of UVB radiation (40 mJ/cm2 UVB) with various amounts of the heat shock exosomes compared to a media control (Media Only) according to one or more embodiments of the present disclosure.

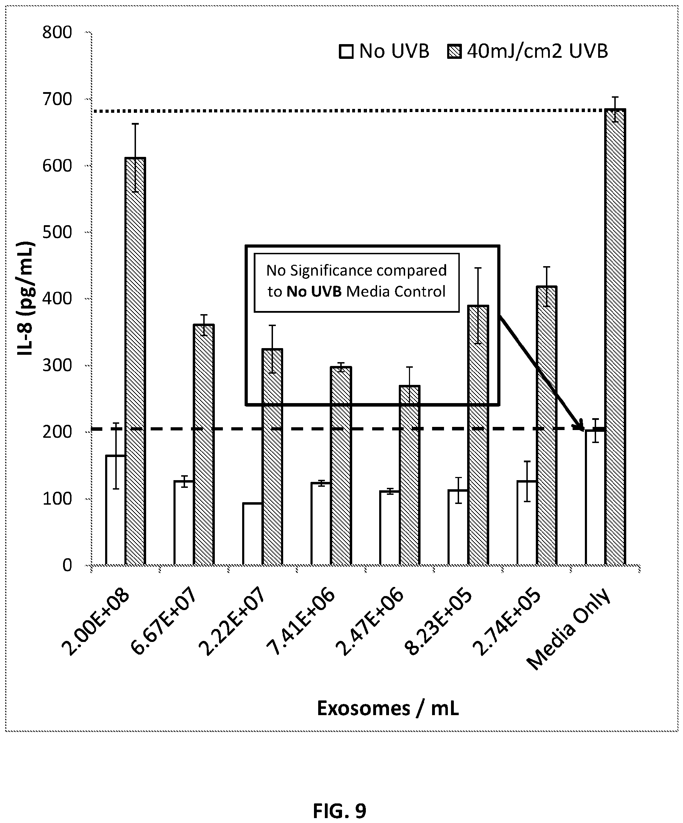

**FIG. 9** is a graph showing a side-by-side comparison of the data in the FIG. 7 and FIG. 8 graphs.

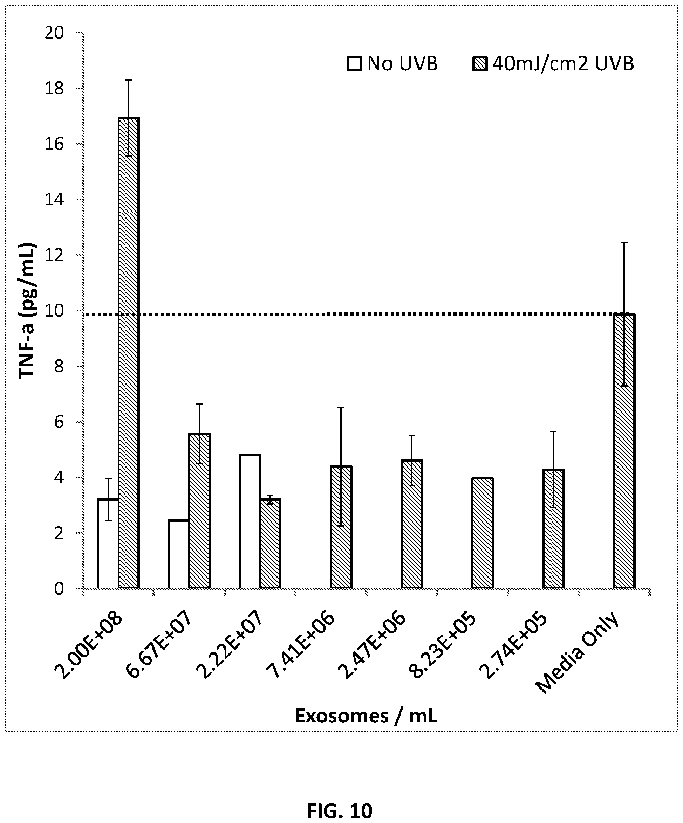

**FIG. 10** is a graph showing the amount of TNF-α produced in the presence of various concentrations of heat shock exosomes in the presence (40 mJ/cm2 UVB) and absence (No UVB) of UVB radiation as compared to a media only control (Media Only) according to one or more embodiments of the present disclosure.

### DETAILED DESCRIPTION

For the purposes of promoting an understanding of the principles of the present disclosure, reference will now be made to preferred embodiments and specific language will be used to describe the same. It will nevertheless be understood that no limitation of the scope of the disclosure is thereby intended, such alteration and further modifications of the disclosure as illustrated herein, being contemplated as would normally occur to one skilled in the art to which the disclosure relates.

There is an unmet need for more effective topical formulations for regulating skin condition such as the treatment and prevention of skin damage, wrinkles, and other defects including scars, keloids, skin discolorations, and skin abrasions. Another important and unmet need remains for more effective formulations to repair soft tissue damage, including repair of periodontal tissue, and repair of burns including burns resulting from radiation treatment. To solve these unmet needs, the presently disclosed subject matter provides improved stem cell-derived exosome compositions, including mesenchymal stem cell (MSC)-derived exosome compositions, and methods for their preparation and use, to regulate skin condition and repair soft tissue damage.

Exosomes represent a compelling therapeutic for a range of indications, especially those requiring delivery to tissues with reduced vasculature or prominent necrosis. Exosomes, unlike stem cells, do not require an oxygenated blood supply to exert their impact. And, because exosomes fuse with cell membranes directly, there is no requirement for receptor mediated uptake of their pro-healing cargos. Accordingly, the isolated exosomes produced according to the methods provided herein can have advantages over existing systemic pharmaceuticals or direct application of stem cells for regulating skin condition and repairing soft tissue damage.

The improved exosome-containing compositions of the present disclosure are based on the context-dependency of the loading of exosomes. More specifically, the present disclosure provides methods demonstrating that exosome loading can be engineered to result in exosomes having enhanced healing activities, such as and including increased proliferative and anti-inflammatory activities. The isolated exosomes of the present disclosure are prepared from stem cell cultures in a highly controlled environment, and various stimuli are delivered to the stem cell cultures to manipulate the exosomal cargo. In one example of providing exosomes engineered for pro-healing activity, stem cell cultures are subjected to high temperature (otherwise known as "heat shock") to produce exosomes having increased levels of heat shock stress-response molecules, including the stress-response protein, HSP70. It is demonstrated herein that the isolated exosomes having increased heat shock stress-response molecules have increased proliferative and anti-inflammatory activity in cell cultures.

The terms "exosomes", "microvesicles", "secreted microvesicles", "extracellular vesicles", and "secreted vesicles" are used interchangeably herein for the purposes of the specification and claims.

The terms "freeze drying" and "lyophilization" are used interchangeably herein for the purposes of the specification and claims.

The terms "stress-response molecules" and "heat shock stress-response molecules" are used interchangeably herein for the purposes of the specification and claims. These terms are meant to include molecules present in exosomes that are secreted by cultured stem cells subjected to high temperature (otherwise known as "heat shock"). Similarly, the terms "exosomes" and "heat shock exosomes" and "heat shocked exosomes" are used interchangeably herein for the purposes of the specification and claims to represent exosomes that are secreted by cultured stem cells subjected to high temperature (otherwise known as "heat shock").

The terms "a," "an," and "the" refer to "one or more" when used in this application, including the claims.

Throughout this specification and the claims, the terms "comprise," "comprises," and "comprising" are used in a non-exclusive sense, except where the context requires otherwise. Likewise, the term "include" and its grammatical variants are intended to be non-limiting, such that recitation of items in a list is not to the exclusion of other like items that can be substituted or added to the listed items.

For the purposes of this specification and claims, the term "about" when used in connection with one or more numbers or numerical ranges, should be understood to refer to all such numbers, including all numbers in a range and modifies that range by extending the boundaries above and below the numerical values set forth. The recitation of numerical ranges by endpoints includes all numbers, e.g., whole integers, including fractions thereof, subsumed within that range. For example, the recitation of about 41 to about 43 includes 41, 42, and 43, as well as fractions thereof, for example, but not limited to, 40.5, 40.6, 40.7, 40.8, 40.9, 41.5, 42.25, 42.5, 43.1, 43.2, 43.3, 43.4, 43.5 and the like, and the recitation of 1 to 3 includes 1, 2, and 3, as well as fractions thereof, for example, but not limited to, 0.6, 0.7, 0.8, 0.9, 1.5, 2.25, 3.5, and the like and any range within that range.

More specifically, in one embodiment of the present disclosure a composition is provided, the composition including: i) isolated stem cell exosomes having increased levels of heat shock stress-response molecules; and ii) a carrier, wherein the stem cell exosomes are produced by a process including: (a) culturing stem cells in a culture medium, wherein the culturing includes a step of heat shocking the stem cells in a serum-free culture media by increasing the culture temperature to about 41°C to about 43°C for about 1 hour to about 3 hours; and (b) isolating the exosomes having increased levels of heat shock stress-response molecules from the serum-free culture medium.

The composition can be in the form of a liquid, lotion, cream, gel, foam, mousse, spray, paste, powder, or solid.

In the composition, isolating the exosomes can be carried out by one or more centrifugation steps. The one or more centrifugation steps can include centrifugation at 100,000X g or greater. In the composition, isolating the exosomes can further include freeze drying the isolated exosomes. In the composition, the process can further comprise culturing the stem cells in the serum-free culture medium at a temperature of about 36°C to 38°C for about 24hr to about 72hr subsequent to the step of heat shocking. The serum-free medium can be free of animal products. The stem cells can be mesenchymal stem cells. The mesenchymal stem cells can be of placental or adipose origin. The heat shock stress-response molecules can include HSP70.

In one embodiment, a method is provided for making stem cell exosomes having increased levels of heat shock stress-response molecules, the method including: culturing stem cells in a culture medium, wherein the culturing includes a step of heat shocking the stem cells in a serum-free culture media by increasing the culture temperature to about 41°C to about 43°C for about 1 hour to about 3 hours, and wherein the serum-free culture medium contains the exosomes having the increased levels of heat shock stress-response molecules.

The method can further include isolating the exosomes from the serum-free culture medium. The isolating can be carried out by one or more centrifugation steps. The one or more centrifugation steps can include centrifugation at 100,000 X g or greater.

The method can further include freeze drying the isolated exosomes, such that the exosomes can be stored at room temperature.

The method can further include culturing the stem cells in the serum-free culture medium at a temperature of about 36°C to 38°C for about 24hr to about 72hr subsequent to the step of heat shocking.

In the method, the serum-free medium can be free of animal products. The stem cells can be mesenchymal stem cells. The mesenchymal stem cells can be of placental or adipose origin. The heat shock stress-response molecules can include HSP70.

Characterization of the size and shape of the isolated exosomes produced according to the methods of the present disclosure is described in Example 3 and the results are shown in the graph in FIG. 1. FIG. 1 shows the size distribution of a representative sample of isolated exosomes with mean of 152 nm and a mode of 107 nm. A scanning electron microscopy (SEM) micrograph from another isolated exosome preparation according to one or more embodiments of the present disclosure is shown in the inset for FIG. 1.

Example 4 describes analysis of the isolated exosomes produced according the methods of the present disclosure for specific protein markers including Hsp70. The resulting data are shown in FIG. 2. FIG. 2 is a bar graph of quantified Western Blot data showing the amount of HSP70 relative to β-actin in the exosomes secreted by stem cells cultured at 37°C without a heat shock step (Control) and exosomes from the same stem cells subjected to a 2 hr heat shock step at 43°C (Heat Shock). The data in FIG. 2 indicate that there is a significant up-regulation in exosomal HSP70 relative to β-actin in the exosomes from the heat shocked cells as compared to the exosomes from the cells cultured without the heat shock step.

The capability of the isolated exosomes prepared according to the methods of the present disclosure to deliver cargo to cells was assessed by monitoring the ability of the isolated exosomes to transfer a lipophilic dye to cells in culture. The experiment is described in Example 5 and the results are shown in FIG. 3. The results indicate an efficient transfer of the dye from the isolated exosomes to the Human pulmonary artery endothelial (HPAE) cells with 75% of the cells being labeled.

The effects of the isolated exosomes produced according to the methods of the present disclosure on cultured periodontal and dermal cells are described in Example 6. FIG's 4A and 4B show that treatment with the isolated exosomes from the heat shocked cells significantly increased proliferation of both periodontal ligament fibroblasts (PDLFs) and dermal fibroblasts (DFs), as compared to the isolated exosomes prepared from cells that were not subjected to a heat shock step. In addition, the level of proliferation of the PDLFs and DFs induced by the isolated exosomes from the heat shocked cells approached or surpassed that induced by complete medium and the individual growth factors.

In addition to degradation of collagen fiber and the extracellular matrix associated with skin aging and its relationship to wound repair, periodontal disease is associated with degradation of the extracellular matrix and collagen fiber degeneration. Additional experiments described in Example 6 demonstrate that the isolated exosomes prepared from heat shocked cells according to the methods of the present disclosure can induce collagen I synthesis in PDLFs and DFs. The graphs in FIG. 5A and 5B show that treatment with the isolated exosomes from the heat shocked cells increased collagen I production of both PDLFs and DFs, as compared to the isolated exosomes prepared from cells that were not subjected to a heat shock step. In addition, the increase in collagen I production of the PDLFs and DFs induced by the isolated exosomes from the heat shocked cells surpassed that of the individual growth factors. These data indicate that the isolated exosomes can have a role in regulating skin condition and repair of soft tissue damage.

*P. gingivalis* is one of the bacterial species known to contribute to periodontitis pathogenesis by secreting various toxins lethal to oral soft tissue cells. Previous reports indicate the induction of inflammatory cascades in gingival keratinocytes (GKs) and PDLFs in response to *P. gingivalis* lysates, including the inflammatory molecules IL6 and IL8 [22-24]. In the experiment described in Example 7, PDLF cells were concomitantly exposed to lyophilized heat killed *P. gingivalis* (HKPG, 10⁷/ml) and the isolated exosomes from medium from heat shocked cell cultures according to the methods of the present disclosure. The results indicate a statistically significant elevation in IL-6 gene expression in HPLF cells induced by heat-killed *P*. *gingivalis* (HKPG) at 1×10^7 /ml. The elevation is significantly reduced by the isolated standard exosomes, and even more so by the isolated cell exosomes produced with a heat shock step. These data indicate that the isolated exosomes of the present disclosure can inhibit the production of inflammatory cytokines including IL6 that act locally to recruit monocytes to the site of inflammation.

A method is disclosed for treating a skin condition, the method including one or more of putting on, embedding into, or filling an area on the skin of a living body a composition of the present disclosure including isolated stem cell exosomes having increased levels of heat shock stress-response molecules, wherein the condition of the skin is treated.

The skin condition can include, for example, one or more of a wound, a burn, a burn resulting from radiation treatment, a discoloration, a scar, and a keloid.

In one embodiment, a topical composition is provided for regulating skin condition, the composition comprising an effective amount of isolated exosomes having increased levels of heat shock stress-response molecules and a carrier.

In one embodiment a topical composition is provided for regulating skin condition, the composition including: i) an effective amount of isolated exosomes having increased levels of heat shock stress-response molecules; and ii) a carrier, wherein the isolated exosomes are isolated from a serum-free culture medium conditioned by culturing stem cells under conditions that include a heat shock of the stem cells in the serum-free culture medium at a temperature of about 41°C to about 43°C for about 1 hour to about 3 hours.

In one embodiment, a topical composition for regulating skin condition is provided, the composition comprising: i) an effective amount of isolated exosomes having increased levels of heat shock stress-response molecules; and ii) a carrier, wherein the isolated exosomes are produced by a process comprising: (a) culturing stem cells in culture medium, wherein the culturing includes a step of heat shocking the stem cells in a serum-free culture medium by increasing the culture temperature to about 41°C to about 43 °C for about 1 hour to about 3 hours, and wherein the serum-free culture medium contains the exosomes having the increased levels of heat shock stress-response molecules; and (b) isolating the exosomes having increased levels of heat shock stress-response molecules from the serum-free medium.

In one embodiment, a method is provided for making a topical composition for regulating skin condition, the method comprising combining an effective amount of isolated exosomes having increased levels of heat shock stress-response molecules with a carrier.

In one embodiment, a method is provided for making a topical composition for regulating skin condition, the method including: combining isolated exosomes having increased levels of heat shock stress-response molecules with a carrier, wherein the exosomes are isolated from a serum-free culture medium conditioned by culturing stem cells under conditions including a heat shock of the stem cells at a temperature of about 41°C to about 43°C for about 1 hour to about 3 hours.

In one embodiment, a method is provided for making a topical composition for regulating skin condition, the method comprising combining an effective amount of isolated exosomes having increased levels of heat shock stress-response molecules with a carrier, wherein the exosomes are produced by a process comprising: (a) culturing stem cells in culture medium, wherein the culturing includes a step of heat shocking the stem cells in a serum-free culture medium by increasing the culture temperature to about 41°C to about 43°C for about 1 hour to about 3 hours, and wherein the serum-free culture medium contains the exosomes having the increased levels of heat shock stress-response molecules; and (b) isolating the exosomes having increased levels of heat shock stress-response molecules from the serum-free medium.

In the compositions provided for regulating skin condition, regulating skin condition can include one or more of inducing increased skin integrity by cell renewal; enhancing water content or moisture of skin; reducing trans epidermal water loss, skin flaking, and scaling; improving skin thickness; enhancing skin tensile properties; reducing the appearance of dermal fine lines and wrinkles; improving skin texture; reducing skin pores size; enhancing skin smoothness; improving skin age spots; improving skin tone; or improving the appearance of scars and skin abrasions.

In the compositions provided for regulating skin condition, the composition can further include from about 0.1 to about 20% of a moisturizing agent. The moisturizing agent can include one or more of panthenol, pantothenic acid derivatives, glycerin, glycerol, dimethicone, petrolatum, hyaluronic acid, or ceremides, and mixtures thereof.

In the compositions provided for regulating skin condition, the composition can further include a vitamin B₃ compound. The vitamin B3 compound can include tocopherol nicotinate.

In the compositions provided for regulating skin condition, the composition can further include an anti-oxidant. The anti-oxidant can include one or a combination of tocopherol or esters of tocopherol.

In the compositions provided for regulating skin condition, the isolated exosomes can be freeze dried.

In one embodiment, a method is provided for regulating a human skin condition which includes applying to human skin at least once a day over at least seven days a topical composition according to the present disclosure comprising isolated exosomes having increased levels of heat shock stress-response molecules. The method can further include applying the topical composition according to the present disclosure to human skin at least twice a day over at least fourteen days.

### EXAMPLES

The following Examples have been included to provide guidance to one of ordinary skill in the art for practicing representative embodiments of the presently disclosed subject matter. In light of the present disclosure and the general level of skill in the art, those of skill can appreciate that the following Examples are intended to be exemplary only and that numerous changes, modifications, and alterations can be employed without departing from the scope of the presently disclosed subject matter.

### Example 1

### Preparation of Exosomes with Increased Levels of Heat Shock Stress-Response Molecules using Heat Shock

The following experiments describe the production of isolated exosomes having increased levels of heat shock stress-response molecules to provide enhanced proliferative and anti-inflammatory activity.

Mesenchymal stem cells (placental or adipose origin) were cultured in a hollow fiber cartridge bioreactor (FIBERCELL BIOSYSTEMS) to produce exosomes having increased levels of heat shock stress-response molecules as follows. Prior to seeding, the bioreactor was conditioned with complete culture medium (DMEM/F12 containing 10% FBS) for 24 hr at 37°C in a humidified, 5% CO₂ containing atmosphere. The bioreactor was seeded with 300 × 10⁶ mesenchymal stem cells (placental or adipose origin) and maintained at 37°C in a humidified, 5% CO₂ containing atmosphere. Cells were grown for 2 weeks before beginning exosome harvest. Prior to harvesting exosome-containing medium, the bioreactor was washed 5 times with serum-free DMEM/F12 to remove bovine exosomes. After washing, the cells were subjected to a heat shock step as follows. The medium in the bioreactor was replaced with serum-free DMEM/F12 medium warmed to 41°C, and the bioreactor was placed in a 41°C, humidified, 5% CO₂ containing atmosphere for 1 hr. Next the 41°C medium was replaced with the same medium warmed to 37°C, and the bioreactor was placed in a 37°C, humidified, 5% CO₂ containing atmosphere for 48 hr. After the 48 hr incubation, the conditioned serum-free DMEM/F12 medium was recovered, and in some instances, stored at -80°C for future processing.

In separate preparations of isolated exosomes having increased levels of heat shock stress-response molecules, the same procedure as described above was followed except that the temperature of the medium used in the heat shock step was about 42°C in some preparations and about 43°C in other preparations and the time period of heat shock ranged from as short as about 1 hour to as long as about 3 hours.

After thawing or fresh collection of the conditioned medium described above, the exosomes were isolated from the conditioned media by centrifugation of the medium at 3000 xg for 20 min at room temperature to pellet cell debris (in 50, 250, or 500 mL screw cap vessels). The clarified supernatant was collected and centrifuged at 100,000 xg (Avg. RCF) for 2 hrs at 4°C. The supernatant was aspirated and the pellet(s) resuspended in minimum volume of DPBS (300-1000 µL). Manufacturer's instructions were followed to estimate protein and RNA concentration using a NANODROP (THERMO FISHER, Corp) spectrophotometer. The number of particles (exosomes) per mL and the particle (exosome) size were determined using the QNANO (IZON SCIENCE, Ltd) following manufacturer's instructions. The isolated exosomes were aliquoted into appropriate volumes into 1.5 mL screw cap tubes.

It was discovered that the isolated exosomes described above could be stored at -80°C and then thawed at a later date for use without a detectable decrease in activity for. It was also discovered that the isolated exosomes could be could be freeze dried and stored at room temperature without a detectable decrease in activity.

### Example 2

### Preparation of Exosomes with Enhanced Proliferative and Anti-Inflammatory Activity using Medium Supplementation

The following experiments describe the production of isolated exosomes having increased proliferative and anti-inflammatory activity.

Mesenchymal stem cells (placental or adipose origin) are cultured in a bioreactor to produce exosomes having increased proliferative and anti-inflammatory activity according to the procedure described above in Example 1 with the following exceptions. After the 2 week period of cell growth, the bioreactor is washed multiple times with serum-free DMEM/F12 to remove bovine exosomes. After washing, the medium in the bioreactor is replaced with serum-free DMEM/F12 medium supplemented with one or a combination of platelet lysate, human platelet lysate, PDGF-BB, TGF-β3, TGF-β1, or other pro- and anti-inflammatory cytokines and the bioreactor is placed in a 37°C, humidified, 5% CO₂ containing atmosphere for 48 hr. After the 48 hr incubation, the conditioned serum-free, supplemented DMEM/F12 medium is recovered and in some instances stored at -80°C for future processing.

After thawing or fresh collection of the conditioned medium described above, the exosomes are isolated from the conditioned media and stored for future use as described in Example 1.

### Example 3

### Size Characterization of Isolated Heat Shock Exosomes

The isolated exosomes produced according to Example 1 were characterized as described in the following experiments.

To determine the size of the stem cell-derived exosomes produced according to Example 1, the isolated exosomes were analyzed using the QNANO (IZON SCIENCE, Ltd) following manufacturer's instructions. The graph in FIG. 1 shows the resulting size distribution of a representative sample with mean of 152 nm and a mode of 107 nm. An exosome sample taken from a separate exosome preparation was analyzed by scanning electron microscopy (MARBLE LABORATORIES) to determine the relative size and shape of the exosome particles. Exosomes were prepared for SEM by drying on mounting studs, coated with platinum, and visualized by SEM (see FIG. 1 inset). While the resulting particle size calculated by SEM was larger than that determined by the QNANO, the difference is likely due to SEM preparation and drying artifacts rather than a significant size variation in the exosome preparations.

### Example 4

### HSP70 is Up-Regulated in Isolated Heat Shock Exosomes

As part of the characterization process, the exosomes prepared according to Example 1 were analyzed by Western blot analysis for specific protein markers including CD63, Hsp70 and TSG101. Specifically, exosomes produced by cells at both normal culture temperature (37°C) (i.e., without a heat shock step) and exosomes produced by cells at culture conditions that include culturing the cells at 43°C for 2 hours according to Example 1 were examined by Western Blot analyses for the presence of stress-response proteins including HSP70. **FIG. 2** is a bar graph of the quantified Western Blot data that shows the amount of HSP70 protein relative to β-actin protein in two separate preparations of exosomes: 1) secreted by cells cultured at 37°C without a heat shock step (Control; blank and hatched bars represent the separate preparations); and 2) secreted by cells subjected to a 2 hr heat shock step at 43°C as described in Example 1 (Heat Shock; blank and hatched bars represent the separate preparations). The data in FIG. 2 indicate that there is a significant up-regulation in exosomal HSP70 relative to β-actin in the heat shock exosomes as compared to the exosomes from the cells cultured without the heat shock step.

### Example 5

### Dye Transfer of Isolated Heat Shock Exosomes to HPAE Cells

The capability of the isolated exosomes prepared according to Example 1 to deliver cargo to cells was assessed by monitoring the ability of the isolated exosomes to transfer a lipophilic dye to cells in culture. The experiments were performed as described below.

An aliquot of isolated exosomes produced according to Example 1 was labeled with VYBRANT DII (LIFE TECHNOLOGIES) cell labeling solution for 20 minutes at 37°C and at 4°C and the dye transfer was assessed at each temperature using flow cytometry [25]. FIG. 3 shows histograms of the data from the cells incubated at 4°C (left-most histogram) and those incubated at 37°C (right-most histogram) with dye-loaded exosomes. The results indicate an efficient transfer of the dye from the exosomes to the human pulmonary artery endothelial (HPAE) cells with 75% of the cells being labeled.

### Example 6

### Effects of Isolated Heat Shock Exosomes on Cultured Periodontal Cells

The effects of the isolated exosomes produced according to Example 1 on cultured periodontal cells were determined as described below.

Adipose-derived stem cell isolated exosomes produced by cells at both normal culture temperature (37°C) (i.e., without a heat shock step) and isolated exosomes produced by cells at culture conditions that included culturing the cells with a heat shock step according to Example 1 were added to low density periodontal ligament fibroblasts (PDLFs) and dermal fibroblasts (DFs) (3,000 cells/well) in 96-well culture plates in serum free medium and incubated for 3 days. To compare the proliferative effects of the isolated exosomes, the cells were also treated with other inducers, including 10% FBS, PDGF, TGF-β1, or IGF-1. After 3 days, the cells were treated with CELL TITER BLUE REAGENT (PROMEGA) for 2 hours to assess proliferation. The data are shown in FIG. 4A (PDLFs) and 4B (DFs). FIG. 4A and 4B show that treatment with the isolated exosomes from the heat shocked cells significantly increased proliferation of both PDLFs and DFs, as compared to the isolated exosomes prepared from cells that were not subjected to a heat shock step. In addition, the level of proliferation of the PDLFs and DFs induced by the isolated exosomes from the heat shocked cells approached or surpassed that induced by complete medium and the individual growth factors.

Periodontal disease is associated with degradation of the extracellular matrix and collagen fiber degeneration. The following experiments were performed to determine if the isolated exosomes prepared from heat shocked cells could induce collagen I synthesis in PDLFs and DFs. For the experiment, isolated exosomes produced by MSC's at both normal culture temperature (37°C) (i.e., without a heat shock step) and isolated exosomes produced by MSC's at culture conditions that included culturing the cells with a heat shock step according to Example 1 were tested along with serum-free conditioned medium from vehicle and growth factors using a procollagen I C-peptide ELISA (TAKARA) assay. PDLF cells were treated for 48 hours with the media control (No Treatment), 20ng/ml TGFβ-1, 10ng/ml IGF, 100ng/ml PDGF, or the isolated exosomes. After the 48 hrs, the conditioned medium was removed, clarified by centrifugation, and diluted into the ELISA assay. The resulting data are shown in FIG. 5A (PDLFs) and FIG. 5B (DFs). The graphs in FIG. 5A and 5B show that treatment with the isolated exosomes from the heat shocked cells increased collagen I production of both PDLFs and DFs, as compared to the isolated exosomes prepared from cells that were not subjected to a heat shock step. In addition, the increase in collagen I production of the PDLFs and DFs induced by the isolated exosomes from the heat shocked cells surpassed that of the individual growth factors. These data indicate that the isolated exosomes can have a role in periodontal ligament repair.

### Example 7

### ASC-Derived Heat Shock Exosomes Inhibit Expression of Inflammatory Cytokines

The potential of ASC-derived isolated exosomes produced according to Example 1 to inhibit IL6 expression in periodontal ligament fibroblasts (PDLFs) was examined as described below.

*P. gingivalis* is one of the bacterial species known to contribute to periodontitis pathogenesis by secreting various toxins lethal to oral soft tissue cells. Previous reports indicate the induction of inflammatory cascades in GKs and PDLFs in response to *P. gingivalis* lysates, including the inflammatory molecules IL6 and IL8 [22-24]. To evaluate the anti-inflammatory impact of treatment with the isolated exosomes prepared from cells cultured with a heat shock step, PDLF cells were concomitantly exposed to lyophilized heat killed *P. gingivalis* (HKPG, 10⁷/ml) and the isolated exosomes from medium from heat shocked cell cultures.

For the experiment, isolated exosomes produced by ASC's at both normal culture temperature (37°C) (i.e., without a heat shock step) and isolated exosomes produced by ASC's at culture conditions that included culturing the cells with a heat shock step according to Example 1 were tested. Specifically, to measure inflammatory response, RT-qPCR for the inflammatory cytokine IL6 mRNA was performed. PDLFs were seeded in 6-well plates and incubated overnight: without HKPG and without exosomes (No Tx), with 10⁷/ml HKPG and without exosomes (No Exosomes), with 10⁷/ml HKPG in combination with adipose stem cell-derived isolated exosomes prepared from cell cultures with a heat shock step (Heat Shocked Exosomes) and without a heat shock step (Std Exosomes). The quantified RT-qPCR data are shown in the graph in FIG. 6. The results indicate a statistically significant elevation in IL-6 gene expression in HPLF cells induced by heat-killed *P. gingivalis* (HKPG) at 1×10^7 /ml. The elevation is significantly reduced by the isolated standard exosomes, and even more so by the isolated cell exosomes produced with a heat shock step. These data indicate that the isolated exosomes of the present disclosure can inhibit the production of inflammatory cytokines including IL6 that act locally to recruit monocytes to the site of inflammation.

### Example 8

### Protective Effect of Heat Shock Exosomes against UVB Light in Human Adult Keratinocytes

The protective effect of MSC-derived isolated exosomes produced according to Example 1 against UVB radiation on human adult keratinocytes was examined as described below.

Solar Ultraviolet (UV) light exposure on skin causes photo aging, sunburn, DNA damages, and carcinogenesis. UVB (290-320 nm) induces erythema and DNA damage such as cyclobutane pyrimidine dimers (CPDs) in the epidermis. UVB radiation also results in inflammation, which can be measured in vitro by proinflammatory mediators e.g., TNF-a, IL-8, and PGE2. Furthermore, UVB could damage cells irreversibly (sunburn cells) which are eliminated by induction of apoptosis.

*In vitro* biological methods provide an excellent tool with which to assess the molecular damage caused by UVB and to evaluate the efficacy of sunscreen products and topical formulations containing chemical or biological technologies in protecting skin from UVB. Human Adult Keratinocyte culture models have been well established as research tools to evaluate the protective effect of small molecules and other formulations, and to overcome the limitations of testing on human subjects.

In this study, human adult keratinocytes were used for the assessment of UVB-induced cell damage and protective activity of the heat shock exosomes described herein in KM-2 media. Media containing the exosomes were applied to keratinocytes for 1 hour then aspirated. PBS was then placed on the keratinocytes and exposed to 40 mJ/cm² UVB. Following exposure, PBS was aspirated and fresh, stock KM-2 media were applied to cells (200 µL). Media were collected at 24 hours. The non-UVB radiated samples underwent the same protocol with the exception of UVB exposure.

The effects of the heat shock exosomes were assessed by measuring reduced production of IL-8 and TNF-α by the cells, and the results are shown in FIG. 7, FIG. 8, FIG. 9, and FIG. 10. Specifically, FIG. 7 is a graph showing IL-8 reduction in the human adult keratinocytes in the absence of UVB radiation (No UVB) with various amounts of the heat shock exosomes compared to a media control. The results show that the heat shock exosomes at all concentrations tested reduced the production of IL-8. In addition, a concentration of 8.23E+05 heat shock exosomes significantly reduced IL-8 production (t-test, 2 tails, unequal variance).

FIG. 8 is a graph showing IL-8 reduction in the human adult keratinocytes in the presence of UVB radiation (40 mJ/cm2 UVB) with various amounts of the heat shock exosomes compared to a media control. The results were similar to the experiment in the absence of UVB where heat shock exosomes at all concentrations tested, with the exception of 2.00E+08, reduced the production of IL-8. Specifically, concentrations of 2.74E+05, 2.47E+06, 7.41E+06, 2.22E+07, and 6.67E+07 heat shock exosomes /mL significantly reduced IL-8 production (t-test, 2 tails, unequal variance).

FIG. 9 is a graph showing a side-by-side comparison of the data in the FIG. 7 and FIG. 8 graphs. The comparison shows that both UVB (40 mJ/cm2 UVB) and Non-UVB (No UVB) exposed samples follow the same trend. Basal levels of IL-8 production (No UVB, Media Only) are 202 pg/mL (marked by the dashed line). Basal levels of IL-8 production in the presence of the UVB radiation (40 mJ/cm2 UVB, Media Only) are 685 pg/mL (marked by the dotted line). Exosome concentrations of 8.23E+05, 2.47E+06, 7.41E+06, and 2.22E+07 exosomes /mL in the UVB exposed cells resulted in no significant difference compared to cells in the No UVB media control. These results demonstrate the protective effect of heat shock exosomes against UVB induced inflammation.

FIG. 10 is a graph showing the amount of TNF-α in the presence of various concentrations of the heat shock exosomes in the presence (40 mJ/cm2 UVB) and absence (No UVB) of UVB radiation as compared to a media only control (Media Only). The data shown that TNF-α release in the UVB exposed samples follow the same trend as observed for IL-8, with a maximum decrease in TNF-α of about 3-fold with heat shock exosomes at a concentration of 2.22E+07 exosomes /mL. The values of TNF-α are at the lower limit of the assay detection, which is why no data are shown for a majority of the No UVB samples.

In summary, the results indicate that IL-8 release from UVB exposed cells treated with heat shock exosome concentrations of 8.23E+05, 2.47E+06, 7.41E+06, and 2.22E+07 exosomes /mL showed no significant difference compared to the No UVB media control, which shows the protective effect of the heat shock exosomes against UVB induced inflammation. In addition, IL-8 release was significantly reduced from cells treated with the heat shock exosomes at concentration of 8.23+E05 exosomes /mL in the absence of UVB radiation as compared to the No UVB media control. Further, TNF-α release from cells treated with the heat shock exosomes was decreased as much as 3-fold. The above results demonstrate the protective effect of heat shock exosomes against UVB induced inflammation.

### References

1. Arch. Dermatol. 138(11):1462-70, 2002.
2. Webber, J., et al., Proteomics analysis of cancer exosomes using a novel modified aptamer-based array (SOMAscan) platform. Mol Cell Proteomics, 2014. 13(4): p. 1050-64. PMID 24505114.
3. Mears, R., et al., Proteomic analysis of melanoma-derived exosomes by two-dimensional polyacrylamide gel electrophoresis and mass spectrometry. Proteomics, 2004. 4(12): p. 4019-31. PMID 15478216.
4. Ogawa, Y., et al., Proteomic analysis of two types of exosomes in human whole saliva. Biol Pharm Bull, 2011. 34(1): p. 13-23. PMID 21212511.
5. Clayton, A., et al., Induction of heat shock proteins in B-cell exosomes. J Cell Sci, 2005. 118(Pt 16): p. 3631-8. PMID 16046478.
6. Du, J., et al., Allogeneic bone marrow mesenchymal stem cell transplantation for periodontal regeneration. J Dent Res, 2014. 93(2): p. 183-8. PMID 24226426.
7. Li, Y., et al., Adult stem cell-based apexogenesis. World J Methodol, 2014. 4(2): p. 99-108. PMID 25332909.
8. Monsarrat, P., et al., Concise review: mesenchymal stromal cells used for periodontal regeneration: a systematic review. Stem Cells Transl Med, 2014. 3(6): p. 768-74. PMID 24744392.
9. Cai, X., et al., Influence of bone marrow-derived mesenchymal stem cells pre-implantation differentiation approach on periodontal regeneration in vivo. J Clin Periodontol, 2015. PMID 25692209.
10. Tobita, M. and H. Mizuno, Adipose-derived stem cells and periodontal tissue engineering. Int J Oral Maxillofac Implants, 2013. 28(6): p. e487-93. PMID 24278946.
11. Iwata, T., et al., Cell sheet engineering and its application for periodontal regeneration. J Tissue Eng Regen Med, 2013. PMID 23881816.
12. Lee, C.S., et al., Adipose stem cell microbeads as production sources for chondrogenic growth factors. J Stem Cells Regen Med, 2014. 10(2): p. 38-48. PMID 25705097.
13.Inukai, T., et al., Novel application of stem cell-derived factors for periodontal regeneration. Biochem Biophys Res Commun, 2013. 430(2): p. 763-8. PMID 23206704.
14. Katagiri, W., et al., Novel cell-free regeneration of bone using stem cell-derived growth factors. Int J Oral Maxillofac Implants, 2013. 28(4): p. 1009-16. PMID 23869359.
15.Osugi, M., et al., Conditioned media from mesenchymal stem cells enhanced bone regeneration in rat calvarial bone defects. Tissue Eng Part A, 2012. 18(13-14): p. 1479-89. PMID 22443121.
16. Kinoshita, Y., et al., Periodontal ligament cell culture on the hydrophobic substrate coated with proteins of periodontal ligament fibroblast-conditioned medium. J Biomater Sci Polym Ed, 1998. 9(5): p. 489-505. PMID 9648029.
17. van Buul, G.M., et al., Mesenchymal stem cells secrete factors that inhibit inflammatory processes in short-term osteoarthritic synovium and cartilage explant culture. Osteoarthritis Cartilage, 2012. 20(10): p. 1186-96. PMID 22771777.
18. Vezina Audette, R., et al., Inflammatory stimuli differentially modulate the transcription of paracrine signaling molecules of equine bone marrow multipotent mesenchymal stromal cells. Osteoarthritis Cartilage, 2013. 21(8): p. 1116-24. PMID 23685224.
19. Maumus, M., C. Jorgensen, and D. Noel, Mesenchymal stem cells in regenerative medicine applied to rheumatic diseases: role of secretome and exosomes. Biochimie, 2013. 95(12): p. 2229-34. PMID 23685070.
20. Zhang, J., et al., Exosomes released from human induced pluripotent stem cells-derived MSCs facilitate cutaneous wound healing by promoting collagen synthesis and angiogenesis. J Transl Med, 2015. 13(1): p. 49. PMID 25638205.
21. Zhou, Y., et al., Exosomes released by human umbilical cord mesenchymal stem cells protect against cisplatin-induced renal oxidative stress and apoptosis in vivo and in vitro. Stem Cell Res Ther, 2013. 4(2): p. 34. PMID 23618405.
22. Liu, J., Y. Wang, and X. Ouyang, Beyond toll-like receptors: Porphyromonas gingivalis induces IL-6, IL-8, and VCAM-1 expression through NOD-mediated NF-kappaB and ERK signaling pathways in periodontal fibroblasts. Inflammation, 2014. 37(2): p. 522-33. PMID 24162780.
23. Morandini, A.C., et al., Toll-like receptor 2 knockdown modulates interleukin (IL)-6 and IL-8 but not stromal derived factor-1 (SDF-1/CXCL12) in human periodontal ligament and gingival fibroblasts. J Periodontol, 2013. 84(4): p. 535-44. PMID 22680301.
24. Morandini, A.C., et al., Periodontal ligament and gingival fibroblasts participate in the production of TGF-beta, interleukin (IL)-8 and IL-10. Braz Oral Res, 2011. 25(2): p. 157-62. PMID 21537641.
25. Deregibus, M.C., et al., Endothelial progenitor cell derived microvesicles activate an angiogenic program in endothelial cells by a horizontal transfer of mRNA. Blood, 2007. 110(7): p. 2440-8. PMID 17536014.

## Claims

1. A topical composition for regulating a cosmetic skin or hair condition, the composition comprising an effective amount of isolated exosomes having increased levels of heat shock stress-response molecules, wherein the isolated exosomes are heat shock exosomes derived from heat shocked mesenchymal stem cells of placental or adipose origin, and a carrier and wherein the heat shock exosomes are produced by a process comprising:
(a) culturing the stem cells in culture medium, wherein the culturing includes a step of heat shocking the stem cells in a serum-free culture medium by increasing the culture temperature to 41°C to 43 °C for 1 hour to 3 hours, and wherein the serum-free culture medium contains the exosomes having the increased levels of heat shock stress-response molecules; and
(b) isolating the exosomes having increased levels of heat shock stress-response molecules from the serum-free medium.

2. The composition of claim 1, wherein the heat shock exosomes have increased levels of HSP70 relative to β-actin in comparison to comparable exosomes derived from mesenchymal stem cells of placental or adipose origin cultured without a heat shock.

3. The composition of any one of claims 1-2, further comprising from 0.1 to 20% of a moisturizing agent, optionally wherein the moisturizing agent comprises one or more of panthenol, pantothenic acid derivatives, glycerin, glycerol, dimethicone, petrolatum, hyaluronic acid, or ceremides, and mixtures thereof.

4. The composition of any one of claims 1-3, further comprising a vitamin B3 compound.

5. The composition of any one of claims 1-3, further comprising tocopherol nicotinate.

6. The composition of any one of claims 1-3, further comprising an anti-oxidant, optionally one or a combination of tocopherol or esters of tocopherol.

7. The composition of any one of claims 1-6, wherein the heat shock exosomes are freeze dried.

8. The composition of any one of claims 1-7, wherein:
(a) the serum- free culture medium is free of animal products; and/or
(b) the composition is in the form of a liquid, lotion, cream, gel, foam, mousse, spray, paste, powder, or solid.

9. The compositions of any one of claims 1-8, wherein the cosmetic skin or hair condition is caused by UVB radiation.

10. A method of making a topical composition for regulating a cosmetic skin or hair condition, the method comprising combining an effective amount of heat shock exosomes according to any one of claims 1-9 with a carrier.

11. A method for regulating a cosmetic skin condition which comprises topically administering a composition according to any of claims 1-9, wherein the cosmetic skin condition comprises skin integrity, water content or moisture of skin, trans epidermal water loss, skin flaking, and scaling, skin thickness, skin tensile properties, the appearance of dermal fine lines and wrinkles, skin texture, skin pores size, skin smoothness, skin age spots, skin tone, or appearance of scars and skin abrasions.

12. The method of claim 11, wherein the heat shock exosomes induce collagen I production of dermal fibroblasts.

13. The method of claim 11, wherein the heat shock exosomes induce proliferation of dermal fibroblasts.

14. The method of claim 11, wherein the heat shock exosomes inhibit expression of inflammatory cytokines.

15. The method of any one of claims 10-14, wherein the cosmetic skin or hair condition is caused by UVB radiation.

## Patentansprüche

1. Topische Zusammensetzung zum Regulieren eines kosmetischen Haut- oder Haarzustands, wobei die Zusammensetzung eine wirksame Menge isolierter Exosomen mit erhöhten Niveaus an Hitzeschock-Stressreaktionsmolekülen, wobei die isolierten Exosomen Hitzeschock-Exosomen sind, die aus Hitzeschock-behandelten mesenchymalen Stammzellen plazentalen oder adipösen Ursprungs stammen, und einen Träger umfasst und wobei die Hitzeschock-Exosomen durch einen Prozess hergestellt werden, der Folgendes umfasst:
(a) Kultivieren der Stammzellen in Kulturmedium, wobei das Kultivieren einen Schritt des Hitzeschockbehandelns der Stammzellen in einem serumfreien Kulturmedium durch Erhöhen der Kulturtemperatur auf 41 °C bis 43 °C für 1 Stunde bis 3 Stunden beinhaltet und wobei das serumfreie Kulturmedium die Exosomen enthält, die erhöhte Niveaus an Hitzeschock-Stressreaktionsmolekülen aufweisen; und
(b) Isolieren der Exosomen mit erhöhten Niveaus an Hitzeschock-Stressreaktionsmolekülen aus dem serumfreien Medium.

2. Zusammensetzung nach Anspruch 1, wobei die Hitzeschock-Exosomen im Vergleich zu vergleichbaren Exosomen, die aus mesenchymalen Stammzellen plazentaren oder adipösen Ursprungs stammen, die ohne Hitzeschock kultiviert wurden, in Bezug auf β-Actin erhöhte Niveaus an HSP70 aufweisen.

3. Zusammensetzung nach einem der Ansprüche 1-2, ferner umfassend 0,1 bis 20 % eines feuchtigkeitsspendenden Mittels, wobei optional das feuchtigkeitsspendende Mittel eines oder mehrere von Panthenol, Pantothensäurederivaten, Glycerin, Glycerol, Dimethicon, Vaseline, Hyaluronsäure oder Ceremiden und Mischungen davon umfasst.

4. Zusammensetzung nach einem der Ansprüche 1-3, ferner umfassend eine Vitamin-B3-Verbindung.

5. Zusammensetzung nach einem der Ansprüche 1-3, ferner umfassend Tocopherolnicotinat.

6. Zusammensetzung nach einem der Ansprüche 1-3, ferner umfassend ein Antioxidans, optional eines oder eine Kombination von Tocopherol oder Estern von Tocopherol.

7. Zusammensetzung nach einem der Ansprüche 1-6, wobei die Hitzeschock-Exosomen gefriergetrocknet sind.

8. Zusammensetzung nach einem der Ansprüche 1-7, wobei:
(a) das serumfreie Kulturmedium frei von tierischen Produkten ist; und/oder
(b) die Zusammensetzung in Form einer Flüssigkeit, einer Lotion, einer Creme, eines Gels, eines Schaums, eines Mousse, eines Sprays, einer Paste, eines Pulvers oder eines Feststoffs vorliegt.

9. Zusammensetzungen nach einem der Ansprüche 1-8, wobei der kosmetische Haut- oder Haarzustand durch UVB-Strahlung hervorgerufen wird.

10. Verfahren zur Herstellung einer topischen Zusammensetzung zum Regulieren eines kosmetischen Haut- oder Haarzustands, wobei das Verfahren ein Kombinieren einer wirksamen Menge von Hitzeschock-Exosomen nach einem der Ansprüche 1-9 mit einem Träger umfasst.

11. Verfahren zum Regulieren eines kosmetischen Hautzustands, umfassend ein topisches Verabreichen einer Zusammensetzung nach einem der Ansprüche 1-9, wobei der kosmetische Hautzustand Hautintegrität, Wassergehalt oder Feuchtigkeit der Haut, transepidermalen Wasserverlust, Hautschuppung und Schuppenbildung, Hautdicke, Hautzugeigenschaften, Auftreten feiner Hautlinien und -falten, Hautbeschaffenheit, Hautporengröße, Hautglätte, Hautaltersflecken, Hautfarbe oder Auftreten von Narben und Hautabschürfungen umfasst.

12. Verfahren nach Anspruch 11, wobei die Hitzeschock-Exosomen die Kollagen-I-Produktion dermaler Fibroblasten induzieren.

13. Verfahren nach Anspruch 11, wobei die Hitzeschock-Exosomen die Proliferation dermaler Fibroblasten induzieren.

14. Verfahren nach Anspruch 11, wobei die Hitzeschock-Exosomen die Expression von inflammatorischen Zytokinen hemmen.

15. Verfahren nach einem der Ansprüche 10-14, wobei der kosmetische Haut- oder Haarzustand durch UVB-Strahlung hervorgerufen wird.

## Revendications

1. Composition topique permettant de réguler un état cosmétique de la peau ou des cheveux, la composition comprenant une quantité efficace d'exosomes isolés présentant des taux accrus de molécules de réponse au stress de choc thermique, lesdits exosomes isolés étant des exosomes de choc thermique dérivés de cellules souches mésenchymateuses d'origine placentaire ou adipeuse ayant subi un choc thermique, et un vecteur et lesdits exosomes de choc thermique étant produits par un procédé comprenant :
(a) la culture des cellules souches dans un milieu de culture, ladite culture comprenant une étape de choc thermique des cellules souches dans un milieu de culture exempt de sérum en augmentant la température de culture à 41°C jusqu'à 43 °C pendant 1 heure à 3 heures, et ledit milieu de culture exempt de sérum contenant les exosomes présentant des taux accrus de molécules de réponse au stress de choc thermique ; et
(b) l'isolement des exosomes présentant des taux accrus de molécules de réponse au stress de choc thermique du milieu exempt de sérum.

2. Composition selon la revendication 1, lesdits exosomes de choc thermique présentant des taux accrus de HSP70 par rapport à la β-actine en comparaison à des exosomes comparables dérivés de cellules souches mésenchymateuses d'origine placentaire ou adipeuse cultivées sans choc thermique.

3. Composition selon l'une quelconque des revendications 1 à 2, comprenant en outre de 0,1 à 20 % d'un agent hydratant, éventuellement ledit agent hydratant comprenant un ou plusieurs composés parmi le panthénol, les dérivés de l'acide pantothénique, la glycérine, le glycérol, la diméthicone, la vaseline, l'acide hyaluronique ou les cérémides, et leurs mélanges.

4. Composition selon l'une quelconque des revendications 1 à 3, comprenant en outre un composé de vitamine B3.

5. Composition selon l'une quelconque des revendications 1 à 3, comprenant en outre du nicotinate de tocophérol.

6. Composition selon l'une quelconque des revendications 1 à 3, comprenant en outre un antioxydant, éventuellement un composé ou une combinaison de tocophérol ou d'esters de tocophérol.

7. Composition selon l'une quelconque des revendications 1 à 6, lesdits exosomes de choc thermique étant lyophilisés.

8. Composition selon l'une quelconque des revendications 1 à 7 :
(a) ledit milieu de culture exempt de sérum étant exempt de produits animaux ; et/ou
(b) ladite composition se présentant sous la forme d'un liquide, d'une lotion, d'une crème, d'un gel, d'un produit moussant, d'une mousse, d'un spray, d'une pâte, d'une poudre ou d'un solide.

9. Compositions selon l'une quelconque des revendications 1 à 8, ledit état cosmétique de la peau ou des cheveux étant provoqué par un rayonnement UVB.

10. Procédé de fabrication d'une composition topique permettant de réguler un état cosmétique de la peau ou des cheveux, le procédé comprenant la combinaison d'une quantité efficace d'exosomes de choc thermique selon l'une quelconque des revendications 1 à 9 avec un vecteur.

11. Procédé permettant de réguler un état cosmétique de la peau qui comprend l'administration topique d'une composition selon l'une quelconque des revendications 1 à 9, ledit état cosmétique de la peau comprenant l'intégrité de la peau, la teneur en eau ou l'humidité de la peau, la perte d'eau transépidermique, l'écaillement de la peau et la desquamation, l'épaisseur de la peau, les propriétés de tension de la peau, l'apparition de rides et de ridules dermiques, la texture de la peau, la taille des pores de la peau, la douceur de la peau, les taches cutanées de vieillesse, le teint de la peau ou l'apparition de cicatrices et d'abrasions cutanées.

12. Procédé selon la revendication 11, lesdits exosomes de choc thermique induisant la production de collagène 1 de fibroblastes dermiques.

13. Procédé selon la revendication 11, lesdits exosomes de choc thermique induisant la prolifération des fibroblastes dermiques.

14. Procédé selon la revendication 11, lesdits exosomes de choc thermique inhibant l'expression des cytokines inflammatoires.

15. Procédé selon l'une quelconque des revendications 10 à 14, ledit état cosmétique de la peau ou des cheveux étant provoqué par un rayonnement UVB.
